# EUROPEAN PATENT APPLICATION

(11) **EP 4 674 342 A2**
(43) Date of publication of application: **07.01.2026**
(21) Application number: 25196417.7
(22) Date of filing: 10.01.2022
(51) Int. Cl.: A61B 5/00

(54) **APPARATUS AND METHOD FOR DETERMINING AND/OR TREATING MICROVASCULAR OBSTRUCTION**

(30) Priority: 11.01.2021 US 202163136174 P
(62) Divisional of application: 22700206.0
(71) Applicant: CorFlow Therapeutics AG, 6340 Baar (CH)
(72) Inventor: SCHWARTZ, Robert S., Minnesota, 55076 (US); ROTHMAN, Martin T., Santa Rosa, 95409 (US); SEGUIN, Jacques, 3780 Gstaad (CH)
(74) Representative: Vossius & Partner Patentanwälte Rechtsanwälte mbB

(57) **Abstract**

Methods and systems are provided for diagnosis and/or treatment of microvascular dysfunction, such as microvascular obstruction (MVO) by injecting volumetric flows into a vessel using an infusion system to arrest or reverse native antegrade flow, such that an equipoise value of volumetric flow rate and corresponding pressure may be determined, which in turn enables calculation of MVO, absolute hydrodynamic resistance, fractional flow reserve, coronary flow reserve, and other physiologic parameters in real-time or near real time.

## Description

### CROSS-REFERENCE TO RELATED APPLICATIONS

This application claims priority to U.S. Provisional Patent Application No. 63/136,174, filed January 11, 2021, the entire contents of which are incorporated herein by reference.

### TECHNICAL FIELD

Methods and devices are provided for the diagnosis and/or treatment of microvascular function and dysfunction (MVD) and other diseases of the microvasculature of organs, including the heart.

### BACKGROUND

Heart attack or acute ECG ST segment elevation myocardial infarction ("STEMI") is caused by sudden occlusion of an epicardial coronary artery, typically by a fibrin and platelet rich clot, with associated embolic plaque and debris. Electrocardiographic signs of acute transmural myocardial infarction (heart attack) are ST segment elevation manifesting across multiple anatomic ECG leads. ST segment elevation is a hallmark of severe coronary artery occlusion or narrowing, which causes ischemic myocardial injury and cell death. Large vessel occlusion often is associated with small vessel severe stenosis or occlusion (referred to as microvascular obstruction or MVO), hemodynamic collapse, clot with embolic debris, and other effects that reduce blood supply. MVO is an independent predictor of late adverse events, including death and heart failure, with no successful MVO therapy identified to date.

Interventional cardiology is proficient at opening severely narrowed or occluded epicardial coronary arteries using catheters, guide wires, balloons, and stents in a cardiac catheterization laboratory. However, microvascular obstruction cannot be diagnosed nor treated in the catheterization laboratory. Moreover, MVO typically cannot be effectively treated even when it is accurately diagnosed.

Heart muscle salvage (i.e., saving muscle from necrosis caused by ischemia) is of critical concern to ensure good long-term outcomes in patients suffering STEMI. A key component of achieving positive long-term outcome requires minimizing the interval between onset of coronary artery occlusion (at home or outside the hospital) and re-opening the occluded artery in a catheterization laboratory. Interventional cardiologists can reduce the duration of artery occlusion time by implementing streamlined and efficient emergency medical systems. The goal of such procedures is to bring STEMI patients to the catheterization laboratory as soon as possible, thereby avoiding long-term STEMI complications. Complications resulting from STEMI and MVO include systolic and diastolic heart failure, arrhythmias, aneurysms, ventricular rupture and multiple other serious complications. These complications can markedly shorten life and/or impose severe limitations on quality of life.

Modern interventional therapy for acute myocardial infarction has matured over time with impressive clinical results. In recent years, heart attack/STEMI death rates at 30 days post event have dropped from more than 30% to less than 5%. This improvement has been achieved by reperfusing the heart with blood as soon as possible after coronary arterial occlusion, which in turn has resulted by streamlining clinical care systems to open coronary arteries in the catheterization lab as rapidly as possible after heart attack onset. Emergency procedures, including stenting and balloon angioplasty, indisputably have improved early and late clinical results of acute heart attack therapy.

However, substantial challenges remain for treating STEMI patients and reducing long-term complications. These problems include heart failure (poor cardiac function and cardiac enlargement), cardiac/ventricular rupture, persistent ischemic chest pain/angina, left ventricular aneurysm and clot, and malignant arrhythmias.

Late heart failure complicates 25-50% of STEMI cases, and consists of poor left ventricular function and damaged myocardium. Heart failure typically worsens as the heart remodels in shape and size, with associated functional loss. Nearly half of all new heart failure in patients under 75 years is linked to STEMI.

Many years investigating STEMI therapy show that opening the epicardial/large coronary artery is insufficient to completely salvage heart muscle and improve long-term patient outcomes. A very common reason for poor late results after heart attack is microvascular obstruction (MVO). MVO is occlusion or severe flow limitation in the small, internal cardiac microvessels. These microvessels are too small and unreachable to stent or be treated with conventional drug/thrombolytic therapy due to microvessels size and number. Thus, despite widely patent epicardial coronary arteries, residual MVO obstructs blood flow to the myocardium, resulting in ischemia and tissue necrosis and severe long-term heart muscle damage.

MVO thus remains a critical frontier in cardiology. Cardiac microvessels comprise small arteries, arterioles, capillaries and venules that are frequently collapsed and filled with cells, clot and debris (platelets, fibrin, and embolic plaque material) during STEMI. Too often, obstructed microvessels (MVO) do not resolve even after stent placement and present serious long-term negative prognostic implications.

MVO is very common in STEMI patients, even though stenting and balloon angioplasty are successful at opening the large epicardial coronary arteries. MVO occurs in more than half of all STEMI patients, even with good blood flow through open the epicardial arteries and newly placed stents.

MVO extent is key to the severity of myocardial damage and patient outcome. MVO may be accurately detected and measured only via cardiac MRI imaging, which identifies MVO location, extent and severity. MRI, however, cannot be performed emergently or during a cardiac catheterization procedure, as it requires patients to be located in a separate imaging area, may require up to 1 hour to complete, and is a separate, expensive procedure.

Important features of MVO may be summarized by the following:
1. MVO and microvascular dysfunction in STEMI are principal causes of early and late major complications after heart attack.
2. Angiographic "no-reflow" or "low-reflow" is caused by MVO, i.e., obstructed microvessels within the heart muscle. MVO in severe cases is characterized angiographically by very slow radiographic contrast filling and flow in the epicardial coronary arteries, as visualized during coronary treatment in the catheterization laboratory. Radiographic contrast filling, however, is only able to diagnose severe no-reflow cases and thus is not able to detect MVO in the majority of the patients.
3. MVO causes myocardial cell injury and death from prolonged ischemia, poor blood flow, and failure to replenish of key metabolic nutrients, such as glucose. MVO microscopic analysis shows collapsed microvessels with red cells, platelets which cause fibrin clot, dead myocardial cells, inflammatory cells, myocyte cell death, and endothelial cell swelling with death, along the obstructed intramyocardial capillaries.
4. Acute MVO manifests as cardiac arterioles and capillaries completely occluded by platelet and fibrin-rich thrombus, platelet-neutrophil aggregates, dying blood cells and embolic debris, and small vessel collapse due to very low intraluminal pressure caused by the proximal vessel occlusion
5. When MVO complicates acute STEMI/myocardial infarction, far greater heart/myocardial damage occurs, and poor ventricular function occurs early.
6. MVO is very common. It occurs in a. roughly 53% of all STEMI and non-ST segment elevation myocardial infarction (NSTEMI) regardless of epicardial flow, b. 90% of Large Transmural STEMI, c. 40% of MI with TIMI III (normal) X-ray visualized flow, and d. MVO is the single most potent prognostic marker of events after controlling for myocardial infarct size.
7. Patients with microvascular obstruction have more late major adverse cardiovascular events (MACE) than those without MVO (45% versus 9%)
8. MVO is the best predictor of acute and chronic cardiovascular adverse outcomes.
9. MVO acutely becomes late fibrous scar and causes poor cardiac function.

MVO cannot be effectively diagnosed and measured in a conventional catheterization laboratory. Moreover, no effective conventional therapies currently are commercially available. Previously proposed therapies all have proved essentially ineffective, and in some cases, dangerous.

Myocardial infarction is by definition cell death and frequently involves myocardial ischemia. Myocardial infarction may cause short, but profound ischemia, which is reversible ("stunning"), chronic ischemia that occurs when myocardial cells are alive but without sufficient oxygen or nutrients to contract normally ("hibernation"); or necrosis and infarction via prolonged ischemia. Infarction typically spreads as a wave, beginning in the endocardium and spreading across the myocardial wall to the epicardium. Each of these events can be characterized by noninvasive imaging and testing, such as nuclear, echo, and PET methods. An exceptionally good test is provided by cardiac MRI, in which gadolinium contrast may be used to visualize the microvascular obstruction.

Myocardial infarction (MI) resulting in microvascular obstruction (MVO) has profound clinical impact. While epicardial coronary arterial occlusion is well known, it has been hypothesized that microscopic/microvascular plugging by thrombus-platelets and fibrin of the microvasculature also occurs. To date, heterotopic platelet aggregation also occurs.

Histopathologic studies show endothelial cell edema, with fibrin and platelet aggregation in both human cases and in animal models. Microvascular plugging also occurs due to red blood cells, white cells and fibrin-platelet aggregates, often not visible to light microscopy, and can be seen via immunostains and EM/SEM/TEM.

MVO is only one of several disorders under larger classification of microvascular dysfunction. Microvascular dysfunction also occurs in patients without epicardial artery occlusion, and encompasses a much larger patient population than the acute coronary occlusion (STEMI) patient group. The effects of occlusion of vessels less than 200 microns in diameter in patients without epicardial artery (vessels larger than 2 mm) occlusion are poorly understood despite years of study and many failed therapeutic strategies.

There is therefore a need in the art for apparatus and methods that can assess microvascular function and dysfunction in the larger MVD patient population. Such apparatus and methods may benefit patients by providing an assessment in real-time or near real-time. There is also a need in the art for apparatus and methods that can diagnose, quantify, and treat microvascular dysfunction, including microvascular obstruction and tissue necrosis/infarction. Still further, there is a need for apparatus and methods that permit assessment, diagnosis and/or treatment of problems in real time or near real-time, permit treatment decisions, and/or allow real time estimation of microvascular injury and ongoing treatment efficacy.

### SUMMARY

Methods and apparatus are provided for assessment, diagnosis and/or treatment of microvascular dysfunction in real time or near real time. In various embodiments, the microvascular dysfunction may include clinical syndromes such as STEMI/NSTEMI, microvascular obstruction, no-reflow, microvascular spasm cardiogenic shock, and other dysfunctional diseases of the microvasculature. The principles of the present invention are applicable to diagnosis and/or treatment of many organs, including the heart, brain, kidney and several other organs. More particularly, non-limiting embodiments include devices and methods to successfully diagnose, restore patency, re-open and preserve flow, and/or limit reperfusion injury in vessels and organs with microvascular dysfunction. Applications include, but are not limited to, therapy for organ systems including the heart (acute myocardial infarction--primary percutaneous coronary intervention (PPCI)), brain (stroke (CVA)), bowel ischemia/infarction, pulmonary emboli/infarction, critical limb ischemia/infarction, kidney/renal ischemia/infarction, liver, peripheral vascular, neurovascular and others.

In accordance with one aspect of the present invention, a system is provided that includes a specialized infusion and sensing catheter for delivering diagnostic and/or therapeutic agents, and a control console. The control console is programmed with specialized algorithms that can be used to diagnose and/or treat microvascular dysfunction by determining physiologic parameters that may be used to predict physiologic events, such as microvascular obstruction, myocardial infarction, and myocardial ischemia. Methods of operating the inventive system to diagnose and/or treat microvascular dysfunction, such as MVO, also are provided.

Systems and apparatus are included that are configured to perform microvascular function assessment in real time or near real time. The inventive systems and apparatus also may be used to diagnose and treat microvascular dysfunction, such as microvascular obstruction (MVO). In accordance with one aspect of the invention, the system and apparatus permit real time diagnosis and treatment using invasive, catheterization methods employing controlled coronary flow infusion (CoFI) techniques capable of rendering accurate predictions of physiologic events.

In accordance with some aspects, a method is provided for determining one or more vascular physiological parameters for a patient. The method may include: advancing a catheter having a lumen into an arterial vessel of the patient, the arterial vessel having antegrade blood flow and the lumen having a distal end; measuring a reference arterial pressure at a location proximal to the distal end of the lumen; delivering a fluid at a first volumetric flow rate through the lumen into the arterial vessel; measuring a first arterial pressure in the arterial vessel, the first arterial pressure corresponding to the first volumetric flow rate; delivering the fluid at a second volumetric flow rate through the lumen into the arterial vessel; measuring a second arterial pressure in the arterial vessel, the second arterial pressure corresponding to the second volumetric flow rate; and/or determining, based on the first volumetric flow rate, the first arterial pressure, the second volumetric flow rate, and the second arterial pressure, an equipoise volumetric flow rate of the fluid at which the pressure proximate to the distal end of the lumen corresponds to the reference arterial pressure.

Microvasculature resistance may be determined based on the reference arterial pressure and the equipoise volumetric flow rate. The first arterial pressure may correspond to one of a first peak systolic pressure, a first peak diastolic pressure, a first RMS pressure, or a first mean pressure. The second arterial pressure may correspond to one of a second peak systolic pressure, a second peak diastolic pressure, a second RMS pressure, or a second mean pressure. The fluid may contain little or no free oxygen. The volumetric flow rate may be determined using a regression analysis.

In accordance with some aspects, a stenosis is disposed in the arterial vessel and the methods/systems may be used for characterizing the stenosis. For example, the systems/methods may include delivering the fluid at the equipoise volumetric flow rate through the lumen into the arterial vessel; measuring a third arterial pressure proximal of the stenosis; and measuring a fourth arterial pressure distal of the stenosis. The stenosis may be characterized based on the third arterial pressure and the fourth arterial pressure. The stenosis resistance may be determined based on the third arterial pressure, the fourth arterial pressure, and the equipoise volumetric flow rate. The fractional flow reserve may be determined based on the ratio of the fourth arterial pressure to the third arterial pressure.

In some embodiments, the fluid is delivered from the catheter into the arterial vessel through an outlet port at a distal end of the catheter. The fluid may be delivered from the catheter into the arterial vessel via a plurality of holes disposed in a distal end of the catheter. The catheter may be balloonless. For example, the catheter may not include a balloon or other expanding elements.

The reference arterial pressure may be a pressure on the proximal section of the catheter. For example, the reference arterial pressure may be aortic pressure.

In accordance with some aspects, apparatus is provided for assessing a patient with a vascular stenosis and/or dysfunction. For example, the apparatus may include a catheter, a pressure sense, a reference pressure sensor, and/or a controller. The catheter may have a distal region sized and shaped to be advanced into an arterial vessel. The catheter may include a lumen for delivering a fluid into the arterial vessel. The pressure sensor may be disposed at the distal region of the catheter to measure a pressure. The reference pressure sensor may be configured to measure a reference pressure at a location proximal to the distal region. The controller may be operatively coupled to the reference pressure sensor and the pressure sensor. The controller may be configured to: cause the fluid to be delivered at a first volumetric flow rate through the lumen into the arterial vessel; measure a first arterial pressure in the arterial vessel while the fluid is delivered at the first volumetric flow rate; cause the fluid to be delivered at a second volumetric flow rate through the lumen into the arterial vessel; measure a second arterial pressure in the arterial vessel while the fluid is delivered at the second volumetric flow rate; and/or determine an equipoise volumetric flow rate at which the pressure corresponds to the reference pressure.

The controller may be further configured to compute microvasculature resistance by dividing the reference pressure by the equipoise volumetric flow rate. The fluid may contain little or no available oxygen. The pressure sensor may be disposed on the catheter. The pressure sensor may be disposed on a guidewire coupled to the catheter. The catheter may be balloonless. For example, the catheter may not include a balloon or other expanding elements.

The controller may be further configured to determine the equipoise volumetric flow rate using regression analysis. The controller may be further configured to cause the fluid to be delivered at the equipoise volumetric flow rate. The pressure sensor may be configured to be advanced from a proximal side of a stenosis to a distal side of the stenosis. The apparatus may include additional pressure sensors such as a second pressure sensor. The pressure sensor and second pressure sensor may be configured to be disposed on opposite sides of a stenosis. The catheter may deliver the fluid through an outlet port disposed at the distal region. The catheter may deliver the fluid through a plurality of holes disposed at the distal region. The reference pressure sensor may be disposed on the catheter at a location proximal to the distal region. The reference pressure sensor may be disposed in the patient's aorta. The controller may measure a third arterial pressure and a fourth arterial pressure (e.g., responsive to signals from the pressure sensor(s)). The third arterial pressure may be measured at a point proximal of a stenosis in the arterial vessel and the fourth arterial pressure may be measured at a point distal to the stenosis. The controller may characterize the stenosis based on the third arterial pressure and the fourth arterial pressure. The controller may determine a stenosis resistance based on the third arterial pressure, the fourth arterial pressure, and the equipoise volumetric flow rate. The controller may determine a fractional flow reserve based on the ratio of the fourth arterial pressure to the third arterial pressure.

Methods for determining microvascular arterial physiological parameter of a patient are provided. These methods may include delivering a fluid into a vessel of the patient at known flow delivery rates, determining the resulting arterial pressures associated with the known delivery rates, determining an equipoise condition, and determining a microvasculature resistance. In some embodiments, these methods include advancing a catheter into an arterial vessel of the patient, delivering a fluid at a first volumetric flow rate into the arterial vessel, and measuring a first arterial pressure in the arterial vessel. Subsequently, fluid may be delivered into the arterial vessel at a second volumetric flow rate and a second arterial pressure is measured. An equipoise arterial pressure and volumetric flow rate then are computed that correspond to cessation of antegrade blood flow through the vessel. At this calculated equipoise point, the native driving pressure is equal to the distal vessel pressure induced by the administered flow, with the result being zero pressure gradient in the proximal vessel segment and hence zero flow. Next, microvasculature resistance is computed by dividing the equipoise arterial pressure by the corresponding infused volumetric flow rate. In some embodiments, the equipoise arterial pressure and corresponding arterial volumetric flow rate and resulting pressure exhibit a substantially linear relationship. Resistance may thus be calculated using linear regression analysis of the flow-pressure parameters. It will be understood that a linear regression analysis may include interpolation and extrapolation.

Methods for determining larger vessel (such as epicardial coronary artery) arterial physiological parameter of a patient also are provided.

The inventive system may include apparatus for assessing microvascular dysfunction of a patient, including a catheter having a pressure sensor, and a controller configured to deliver fluid through the catheter, wherein the controller is configured to calculate an equipoise pressure value based on at least two fluid flow rates and pressures. In some embodiments, the apparatus may include a catheter having a distal end, a lumen for delivering a fluid into a blood vessel having a blood flow, a pressure sensor disposed near a distal end of the catheter, and a controller programmed to deliver the fluid to the catheter at a plurality of flow rates. The controller is programmed to calculate an equipoise volumetric fluid flow rate and pressure based on the injected fluid flow rates and resulting pressures, and to calculate a value of which matches Cardiac MRI MVO measurement. The catheter may, but need not, include an occlusion balloon. In accordance with one aspect of the invention, the computed MVO is useful since it yields MVO values comparable to those determined by MRI.

### BRIEF DESCRIPTION OF THE DRAWINGS

The foregoing and other objects, features, and advantages of the present disclosure set forth herein will be apparent from the following description of particular embodiments of those inventive concepts, as illustrated in the accompanying drawings. It should be noted that the drawings are not necessarily to scale; emphasis instead is placed on illustrating the principles of the inventive concepts. Also, in the drawings, like reference characters may refer to the same parts or similar parts throughout the different views. It is intended that the embodiments and figures disclosed herein are to be considered illustrative rather than limiting.
FIG. 1 is a perspective view of an exemplary modular system for assessment of MVD in accordance with principles of the present invention;
FIGS. 2A-2C are perspective views of distal ends of alternative embodiments of infusion catheters constructed in accordance with the present invention;
FIG. 3A is perspective view of a central portion of an infusion catheter suitable for use with the distal ends depicted in FIGS. 2A-2D;
FIGS. 3B and 3C are, respectively, a side sectional view and perspective view of an alternative embodiment of a distal portion of an infusion catheter having a pressure chamber;
FIG. 4 is a side view of a distal end of a preferred embodiment of a catheter having one or more pressure sensors/transducers;
FIG. 5 is a schematic view of a system constructed in accordance with the principles of the present invention;
FIG. 6A is a graph depicting an exemplary relationship between infusate pump flow and resulting pressure at the infusate delivery site;
FIG. 6B is a graph depicting an exemplary relationship between native flow rates and infusate flow rates that may be used to demonstrate an equipoise flow rate and pressure in accordance with the present invention;
FIG. 7 is an exemplary electrical circuit model depicting the resistances of the physiologic components embodied in the algorithms of the methodology for calculating MVD in accordance with the principles of the present invention;
FIG. 8 is a flow chart for generating estimates of physiologic parameters employed in computing MVO;
FIG. 9 is a flow chart for determining coronary flow reserve (CFR) in accordance with the principles of the present invention;
FIG. 10 is side view of a distal end of an infusion catheter positioned in a vessel with a stenosis in accordance with embodiments of the present invention;
FIG. 11 is a flow chart for computing stenosis resistance in large epicardial arteries, fractional flow reserve (FFR) and other physiologic hydraulic vascular parameters and function in accordance with the principles of the present invention; and
FIG. 12 is a schematic diagram of components that may be utilized in a system of the present invention.

### DETAILED DESCRIPTION

The present invention is directed to devices, systems and methods for implementing techniques to determine parameters for predicting physiologic events, and is applicable to diagnosis and/or treatment of many organs, including the heart. More particularly, the inventive systems and methods enable successful prediction of physiologic events, such as microvascular obstruction, myocardial infarction, and myocardial ischemia. Applications of the inventive system and methods include diagnosis and treatment of the heart (acute myocardial infarction-primary percutaneous coronary intervention (PPCI)), brain (stroke (CVA)), bowel ischemia/infarction, pulmonary emboli/infarction, critical limb ischemia/infarction, renal ischemia/infarction, liver, peripheral vascular, neurovascular and others obstruction (MVO) and tissue necrosis/infarction.

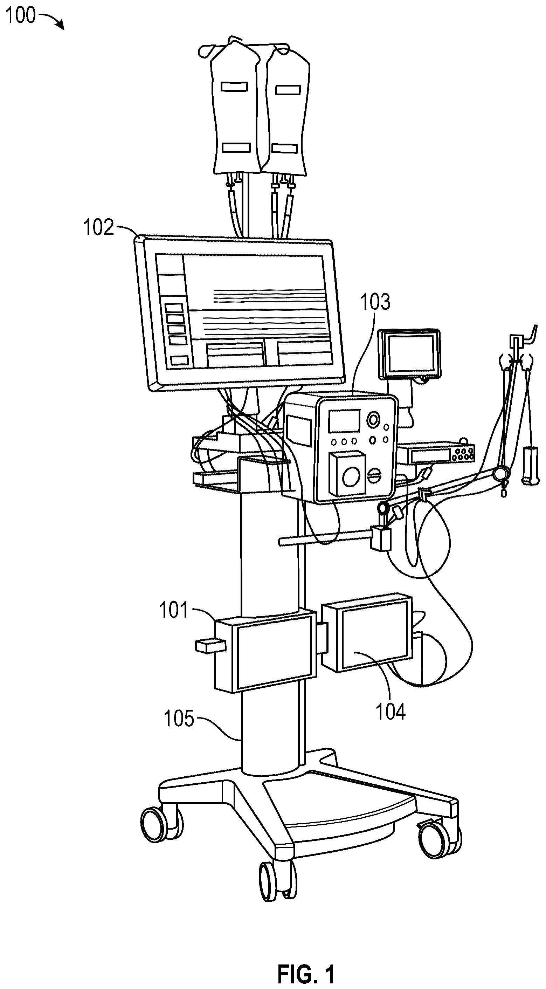

Referring to FIG. 1, exemplary modular computerized diagnostic and infusion system 100 for coronary and other human/animal vasculature and organs is described. Infusion system 100 illustratively is packaged as a mobile console and is programmed to enable characterization of physiologic system and events, including real-time or near real time measurement of coronary artery pressure and flow; pressure/flow time parameters; coronary physiology measures such as coronary flow reserve (CFR) measurements; fractional flow reserve (FFR) measurements; autoregulation function, hemodynamic resistance determinations; equipoise flow determinations; and/or equipoise pressure determinations. Infusion system 100 includes controller 101, display 102, infusion pump 103 and pressure measurement system 104, all mounted on mobile platform 105.

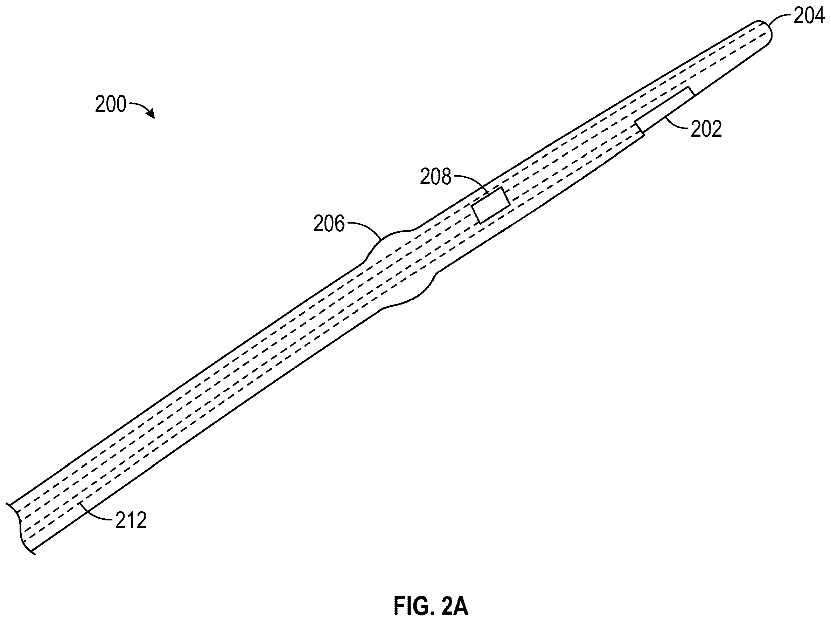

Referring now to FIG. 2A, a distal end of exemplary infusion catheter 200 is described. Infusion catheter 200 includes infusion port 202 in fluid communication with infusion lumen 212 that extends from a proximal of the catheter and couples to infusion pump 103. Guidewire lumen 204 extends through catheter 200 to permit the infusion catheter to be advanced along a guidewire to a desired position within a patient's vasculature. Catheter 200 may have flared an enlarged diameter section 206, which serves to increase hydrodynamic resistance for diagnostic purposes. When exposed to fluid flow, section 206 creates a hydrostatic pressure gradient within a bodily lumen, as explained in U.S. Patent Application Serial No. 17/327,433, published as U.S. Patent Application Publication No. 2021/0361170 and assigned to CorFlow Therapeutics AG, the entire contents of which are incorporated by reference. Pressure sensor 208 is located in the body of catheter 200 and may be coupled to pressure measurement system 204 to determine the pressure of a fluid flow within a patient's vasculature.

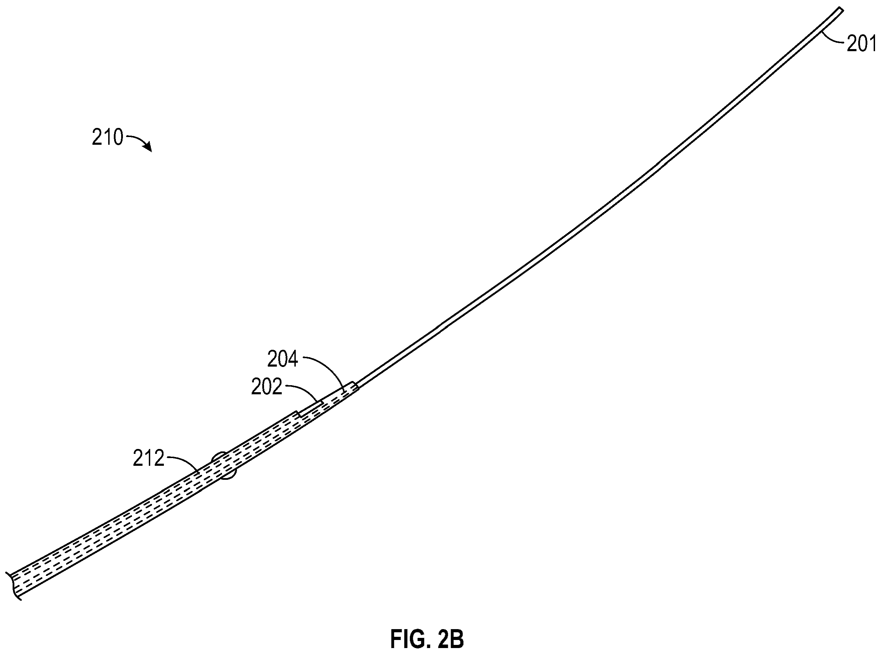

With respect to FIG. 2B, a distal end of an alternative embodiment of an infusion catheter, catheter 210, is described. Guidewire 201, which may be a 0.014" pressure measuring guidewire, may be inserted through catheter 210 in a rapid-exchange (RX) manner so that a distal end of guidewire 201 extends from the exit of guidewire lumen 204. Infusion port 202 is located on the distal end of catheter 201 adjacent to the exit of guidewire lumen 204 deliver fluids via infusion lumen 212, shown in dotted lines.

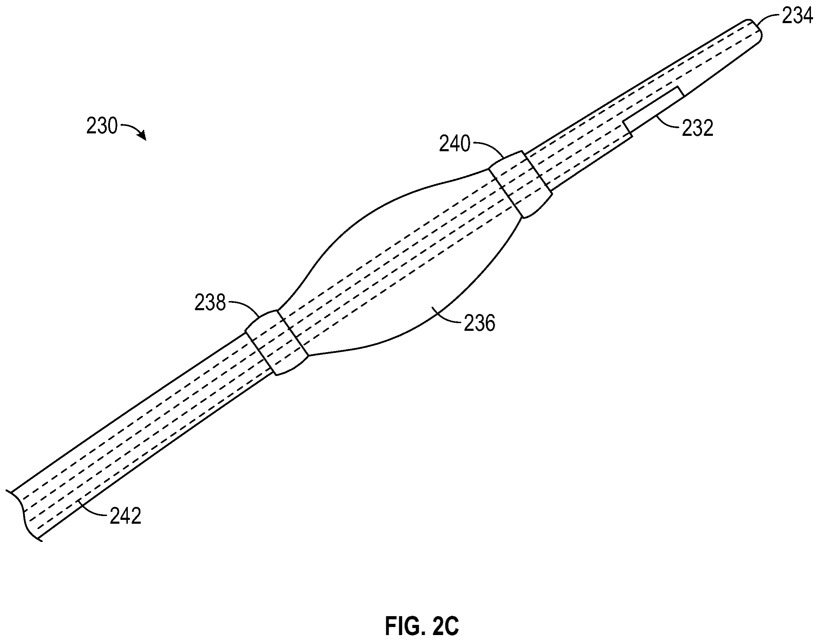

In FIG. 2C, a distal end of another alternative embodiment is described, in which infusion catheter 230 has occlusion balloon 236, radio-opaque markers 238 and 240. Infusion outlet port 232 is disposed in fluid communication with infusion lumen 242, shown in dotted lines. Guidewire lumen 234 also is provided and may be arranged for either over-the-wire or rapid exchange use. Additional embodiments of infusion catheters may be used herein, such as those described in U.S. Patent No. 10,315,016 to Schwartz or U.S. Patent Application Publication No. 2018/0280172 to Hoem, the entire contents of each of which are incorporated herein by reference.

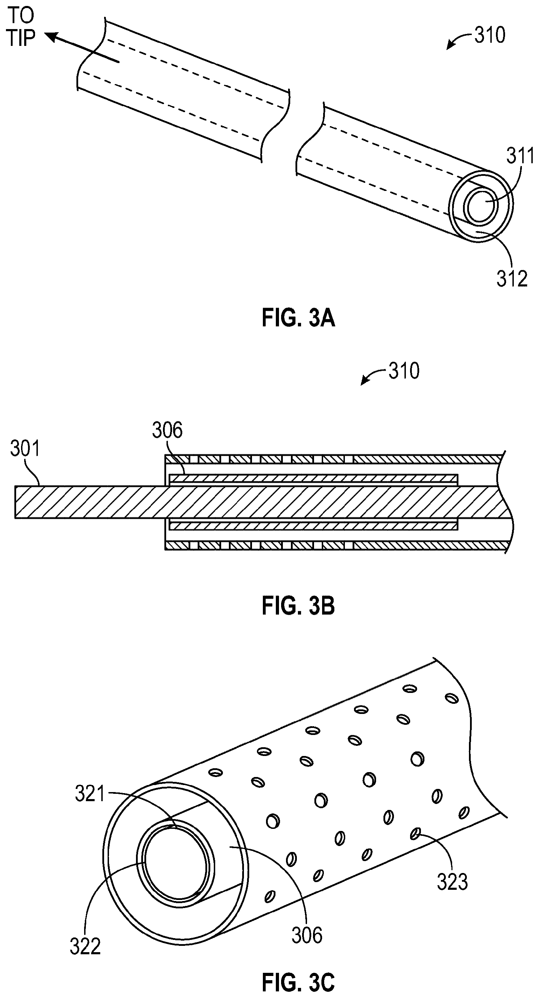

Referring now to FIG. 3A, a central portion of an infusion catheter constructed in accordance with principles of the present invention is described. The central portion of catheter 310 includes infusion lumen 312 that encircles guidewire lumen 311, illustratively co-axially. In alternative embodiments, infusion lumen 312 may be arranged side-by-side, or otherwise, with respect to guidewire lumen 311. In preferred embodiments, the diameter of catheter 310 is small, thereby allowing the catheter to be introduced into smaller vessels for diagnosis and therapy. Guidewire lumen 311 preferably has a diameter sufficient to accept a guide wire that provides pressure sensing.

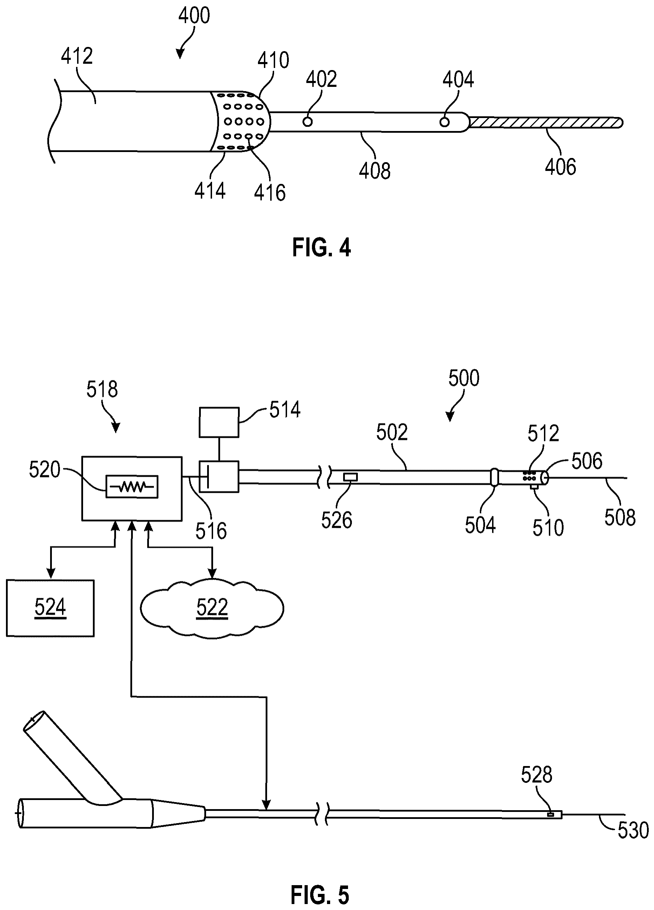

In FIGS. 3B and 3C, a distal portion of an exemplary infusion catheter having pressure chamber 306 disposed on guidewire 301 is described. Pressure chamber 306 is designed to provide a region of stable pressure measurement in a distal arterial segment, and permits pressure measurement at locations different than near or distal to the catheter tip. As shown in FIG. 3C, the exterior surface of the catheter may include pores, slits or slots 323 to provide better dispersion of infusate at the distal end of the catheter and also more precise pressure measurement. Pores, slits, or slots 323 also be arranged to direct infusate flow pattern, as may be desired for a particular type of diagnosis or therapy. Additional catheter configurations for infusing fluid into a vessel, including mechanisms for centering a distal end of the catheter within a vessel to evenly distribute flow, and to direct infused flow preferentially in antegrade and/or retrograde directions within the vessel, are described with respect to FIGS. 5A-7E of copending, commonly assigned U.S. patent application Serial No. 16/577,962, filed September 20, 2019, and published as U.S. Patent Application Publication No. US2020/0093991 A1, which is incorporated herein by reference.

Referring now to FIGS. 4 and 5, an exemplary embodiment of infusion system constructed in accordance with the present invention is described, which includes one or more pressure sensors. FIG. 4 depicts distal end 400 of a preferred embodiment of a catheter that includes distal pressure sensors/transducers 402 and 404. Pressure sensors/transducers 402 and 404 are preferably mounted apart a distance greater than 1 cm longitudinally, and more preferably are spaced apart a distance to permit the sensors to be located on either side of a typical stenosis. Guidewire 406 is disposed in guidewire lumen 408. Distal end 410 of catheter body 412 includes cap 414 containing a plurality of holes 416, in communication with the infusion lumen, through which infusate may be controllably released. The pressure sensor configuration shown in FIG. 4 permits measurement of axial intravascular (longitudinal) pressure gradients. Longitudinally measured pressure gradients may be used to understand the relationship between vascular/arterial intake size and flow, and thus permit assessment of large vessel resistance in which the catheter lies.

For example, fractional flow reserve (FFR) is a parameter that enables assessment of the hemodynamic significance of a coronary artery stenosis, and is typically determined at maximum vasodilation. The two pressure sensor/transducer configuration of FIG. 4 facilitates this measurement without the need for maximum vasodilation. Coronary flow infusion using an infusion pump and pressure measurement also allows simple and direct absolute stenosis and hemodynamic/hydraulic quantitation. Other important physiologic parameters similarly may be measured, including coronary flow reserve (CFR), and microvascular resistance as described below. Fluid infusions without oxygen content such an electrolyte solution avoid the need to pharmacologically induce maximum vasodilation using an intravenous or arterial vasodilation agent such as adenosine. This is due to an induced oxygen-poor state with subsequent physiologic vasodilation. In addition, this sensor configuration shown in FIG. 4 allows absolute hydrodynamic resistance to be quantified, which in turn permits fractional flow reserve calculation using mathematical transformation methods.

With respect to FIG. 5, system 500 constructed in accordance with principles of the present invention, as may be embodied in the modular mobile system depicted in FIG. 1, is described. System 500 includes catheter 502 having a focal or general diameter increase, forming bump 504 and distal end 506. Catheter 502 may be advanced into a desired location in a patient's vascular over pressure-sensing guidewire 508. Catheter 502 may be constructed with an internal lumen to accept guidewire 508 in an over-the-wire or rapid exchange modality. Pressure sensor 510 and infusion holes 512 are disposed near distal end 506. Infusate located in reservoir 514 is selectively pumped through catheter 502 via infusate lumen and exits through infusion holes 512 by pump 516. Controller 518, which may include a processor and programmed algorithms, is in communication with pressure sensor 510 and pressure guidewire 508 and is configured to receive pressure readings from those devices.

Controller 518 also is in communication with pump 516 and is configured to control the flow rate of infusate injected via pump 516. Feedback from calculations of equipoise flow rates, as described below, may be used to adjust the pump, balloon pressure (in embodiments having a balloon present), or operation of other system components to make desired changes in system function and improve diagnostic or therapeutic system function. Controller further includes storage medium 520, which may include RAM, ROM, disk drive, or other known storage media. In some embodiments, storage medium 520 may store algorithms and mathematical calculations disclosed in this application. In some embodiments, storage medium 520 is used to store data that is received from pressure sensor 510 and pressure guide wire 508, as well as to store the equipoise pressure and volumetric flow values, FFR, and other calculated values. In some embodiments, storage medium 520 may include a machine-learning algorithm that controls flow rates, performs measurements, and calculates results. In some embodiments, controller 518 may communicate with external wide area networks, such as Internet 522 and/or computing device 524 to communicate data that may be used to refine algorithms in storage medium 520. Controller 518 also may be programmed to access a database of MRI images and parameters derived from those images used to assess microvascular obstruction dysfunction that can be correlated to MVO values computed using the flow analyses methods of the present invention.

It is desirable to have a reference pressure within a patient's vasculature, which in preferred embodiments is a proximal arterial reference pressure, and in some embodiments may be the aortic pressure. The reference pressure (as well as other pressures referenced in this specification and in the figures) may correspond to a peak systolic pressure, a peak diastolic pressure, a mean pressure, a pressure determined using a root mean square (RMS) method, other types of known pressure measurements, or a combination of any of these types of pressure measurements. System 500 optionally may further include pressure sensor 526 located a proximal distance from distal end 506. In some preferred embodiments, pressure sensor 526 is located sufficiently proximal to distal end 506 that readings taken with pressure sensor 526 may represent an proximal arterial reference pressure. In such embodiments, infusate flow from distal end 506 has a negligible effect, if any, on pressure measurements taken by pressure sensor 526. Alternatively, or in addition to pressure sensor 526, system 500 may include pressure sensor 528 or pressure sensor 530 attached to a second catheter, guidewire, or other delivery device. In some embodiments, pressure sensor 528 is configured to be disposed at a different location than catheter 502 and is used to determine a reference pressure. In still other embodiments, system 500 may be in communication with one or more remote pressure sensors, which are used to determine a reference pressure. A proximal arterial reference pressure may also be obtained using other methods, such as those explained in U.S. Patent Application Serial No. 17/327,433, published as U.S. Patent Application Publication No. 2021/0361170 and assigned to CorFlow Therapeutics AG, the entire contents of which are incorporated by reference.

In preferred embodiments, the infusion catheters as shown in FIGS. 2-5 may be in communication with a controller that is configured to control a pump, such that the pump provides the infusate via an infusate lumen at a known flow rate or flow rates. Controller is also in communication with one or more pressure sensors, which may be located on a guidewire or elsewhere on the catheter. As discussed in greater detail below, controller is configured to determine an equipoise arterial pressure and equipoise volumetric flow rate at which antegrade blood flow ceases. One of skill in the art will appreciate that the disclosed invention will provide apparatus and methods for reducing or stopping antegrade blood flow through a controlled release of infusate.

In one preferred embodiment, system 500 employs an infusion catheter as described with respect to FIG. 4, while in other embodiments the infusion catheters depicted in FIGS. 2 or 3, or as described in the above-incorporated U.S. Patent Application Publication No. US2020/0093991 A1 may be employed. System 500 may also employ infusion catheter as described in U.S. Patent Application Serial No. 17/327,433, which is incorporated by reference. Preferably, an infusion catheter suitable for use with the system 500 is compatible with a 6F guide sheath, includes a guidewire that accepts a 0.014" pressure guidewire, and is capable of infusing volumetric flow rates in a range of 5 to 50 ml/min. Optionally, the infusion catheter may include a compliant or semicompliant occlusion balloon.

In accordance with the principles of the present invention, the infusion catheter is inserted into an epicardial vessel that supplies blood to a patient's myocardium to assess whether the myocardial vessels distal to or nearby vessels manifest microvascular dysfunction, such as MVO and/or may include dysfunctional vessels responsible for myocardial infarction or ischemia. Volumetric flow is introduced through the infusion holes of the catheter at sequentially increased flow rates to block antegrade blood flow in whole or in part within the epicardial or intramyocardial vessel.

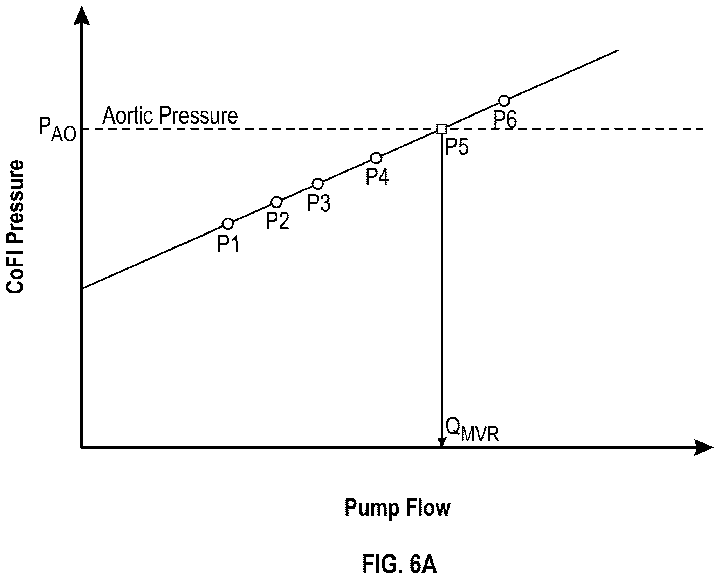

A proximal arterial reference pressure is now discussed with reference to FIG. 6A, in which the proximal arterial reference pressure is represented as aortic pressure, P_{AO}. A proximal arterial reference pressure may be determined in a variety of ways known by those of skill in the art, such as by obtaining an aortic pressure using a sensor disposed in communication with a patient's aorta. Additionally, proximal arterial reference pressure may be determined using pressure sensor 526. Proximal arterial reference pressure could also be determined by taking readings with a distal pressure sensor, such as pressure sensor 510, before advancing that sensor distally, as may occur as catheter 502 is being advanced to the treatment site. It will be appreciated that these two methods may not result in identical determinations of proximal arterial reference pressure, as the latter method may not take into account the increased resistance from the catheter once it is advanced to the treatment site.

Once a catheter in accordance with the present invention is placed at or near the treatment site, distal pressure measurements may be obtained as infusate is provided at one or more flow rates. For example in reference to system 500, catheter 502 may be advanced to the treatment site. The clinician may cause system 500 to deliver an infusate at a first flow rate. As the infusate is being delivered to the patient, one or more pressure measurements may be taken with pressure sensor 510 and compared to one or more pressure measurement taken with sensor 526 representative of an proximal arterial reference pressure. Continuing the example, at the first flow rate, the pressure determined using pressure sensor 510 may be determined to be pressure P₁ and the proximal arterial reference pressure determined using pressure sensor 526 may be determined to be P_{AO}, each of which are depicted on the chart of FIG. 6A, wherein P₁ is shown to have a lower value than P_{AO}. The operator or an automated control system may then increase the flow rate of the infusate to a second flow rate and make a determination of the pressure at pressure sensor 510, which is illustrated as pressure P₂ in FIG. 6A. It will be appreciated that the infused flow rate may continue to increase and the operator or automated control system may determine the associated pressures using pressure sensor 510, and such pressure determinations may be illustrated in FIG. 6A as pressures P₃, P₄, P₅, and P₆. Though preferred embodiments of the invention involve an increase in flow rates (which may, but need not, be stepwise at a fixed amount) from the determination of one pressure to the determination of the next pressure, it will be appreciated that embodiments may lower the flow rate to determine a subsequent pressure after determining a prior pressure at a higher flow rate.

It is desired to determine a flow rate of the infusate that results in a pressure at the infusate delivery site that is the same as, or sufficiently close enough to, the proximal arterial reference pressure (which may be an aortic pressure). In the example shown in FIG. 6A, at flow rate Q_{MVR}, the corresponding pressure P₅, is the same as proximal arterial reference pressure P_{AO}. It may be assumed that flow rate Q_{MVR} represents the flow through microvasculature or flow through a stenosis.

In some embodiments, the operator or system may adjust the infusate flow rate higher or lower until the pressure measurement taken with pressure sensor 510 is sufficiently close to the proximal arterial reference pressure. In the example of FIG. 6A, the system may have taken multiple measurements at different flow rates resulting in pressures between pressure P₁, and pressure P₆ before identifying the flow rate that results in pressure P₅, which is the same as proximal arterial reference pressure P_{AO}. Such a determination may be made solely on a comparison between the pressure determined at the treatment site and the proximal arterial reference pressure.

In some embodiments, it is desirable to determine pressure P₅, corresponding to proximal arterial reference pressure P_{AO} without the need to resort to determining the pressures corresponding to different flow rates in a trial and error process. In this regard, the inventors have found that a linear relationship exists between the infusate flow rate and the pressure determined at the site where the infusate is being delivered. In light of this linear relationship, a clinician may determine a pressure at a first flow rate and then the pressure at a second flow rate, and then use these pressures to determine the flow rate corresponding to the proximal arterial reference pressure mathematically using linear regression. For example, in reference to FIG. 6A, a clinician may determine pressure P₄ occurs at one flow rate and pressure P₆ occurs at another flow rate. Using linear regression, the clinician may determine that pressure P₅ (corresponding to proximal arterial reference pressure P_{AO}) occurs at flow rate Q_{MVR}. Thus, the desired flow rate may be found after making pressure determinations at just two different flow rates.

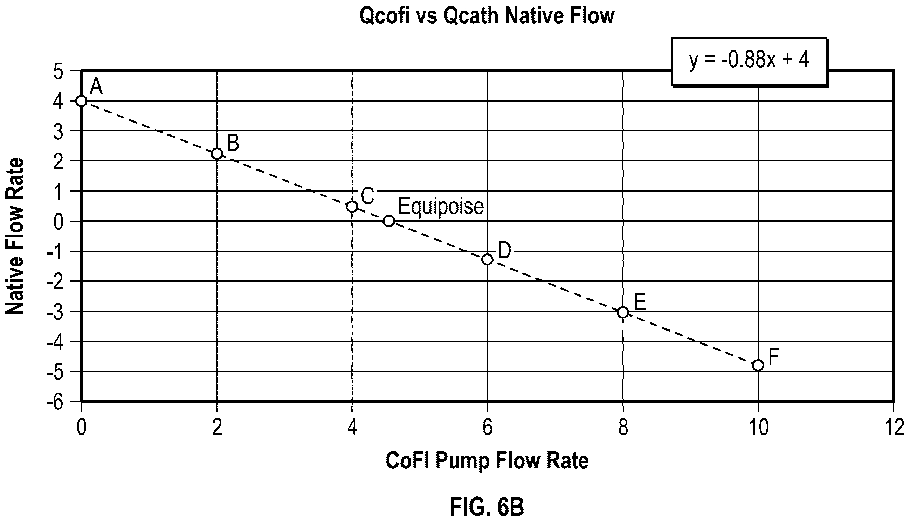

A clinician may want to avoid delivering the infusate at or above a rate that results in a pressure at the delivery site that exceeds the proximal arterial reference pressure. Utilizing linear regression, a clinician in preferred embodiments may determine the resulting pressure at the infusate delivery site for two difference infusate flow rates, each of which results in pressures lower than the proximal arterial reference pressure. For example, in reference again to FIG. 6A, the infusate is delivered at two relatively low infusate flow rates, resulting in pressure P₁ and pressure P₂ at the infusate delivery site. Linear regression may be used to extrapolate the line between points in FIG. 6A to reach point P₅, which corresponds to infusion flow rate Q_{MVR}. Referring now to FIG. 6B, a method is described for determining an equipoise volumetric flow rate and corresponding arterial pressure that may be used to compute microvascular resistance, large vessel vascular or stenosis resistance, , FFR and CFR. In particular, FIG. 6B illustrates a relationship between controlled flow infusion ("CoFI") flow and resulting native vessel flow. The infusate flow is provided by apparatus and methods of the present invention. In FIG. 6B, an equipoise flow is reached when infusate flow into the distal myocardium creates a pressure equal to the native driving pressure. At this point, the net driving pressure gradient is zero, and thus the native proximal flow is zero. As the infusate volumetric flow rate provided by system 500 is increased, which may in some embodiments be in a step-wise manner, the increased arterial pressure resulting from the infusate flow causes a corresponding decrease in native antegrade blood flow rate. For example, at point C, when the infusate volumetric flow rate has a value of 4 ml/min, the native volumetric flow rate falls to a value of approximately 0.5 ml/min. As the infused volumetric flow rate further increases, the native antegrade blood flow stops and then a retrograde (reversed) direction if distal pressure becomes large enough to invert the gradient. Negative values of the native flow rate depicted in the chart of FIG. 6B are associated with retrograde flow. The infusate volumetric flow rate at which antegrade flow ceases is referred to herein as the "equipoise" condition; in FIG. 6B, this equipoise condition occurs when the infusate volumetric flow rate is about 4.5 ml/min.

Applicant discovered that the relationship between the infusate volumetric flow rate and the native volumetric flow rate has a substantially inverse linear relationship. In the example depicted in FIG. 6B, for the specific arterial vasculature tested, the correlation between the native volumetric flow rate and infusate flow rate was determined to be approximated by the equation y = (-0.88)x + 4, where y is the value of the native volumetric flow rate in milliliters/min and x is the value of the infusate volumetric flow rate.

In accordance with aspects of the present invention, the relationship between the volumetric infusate flow rates and measured vascular pressures may be used to determine the equipoise condition. Based on the measured substantially linear relationship between those volumetric flow rates and pressures in both animals and humans, the equipoise condition may be determined using as few as two data points and linear regression analysis. For example, using any two of points P₁ - P₆ in the chart of FIG. 6A and the equation for a line (y = mx + b), the equation may be solved to find the infusate volumetric flow rate corresponding to the reference pressure that results in zero driving pressure gradient and hence zero native volumetric flow rate in the proximal vessel segment. Methods of determining fractional flow reserve (FFR), absolute myocardial resistance, microvascular resistance, and other parameters are described in the above-incorporated U.S. Patent Application No. 16/577,962. For example, as described in that application, distal microvascular resistance related to MVO may be computed by dividing the equipoise pressure by equipoise volumetric flow rate, and MVO thus estimated.

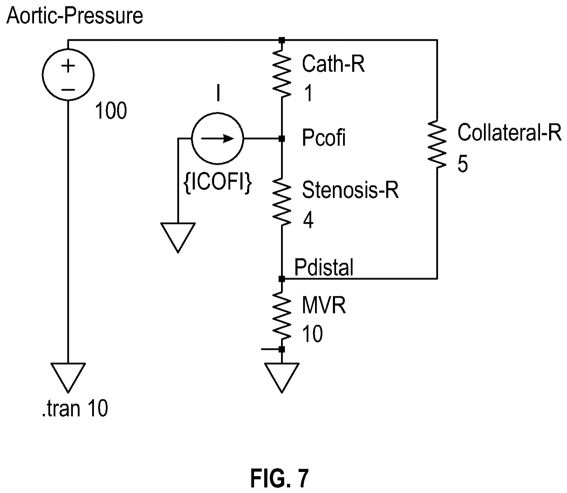

FIG. 7 is a schematic describing a model in accordance with some embodiments of the present invention, which may be used to assess a stenosis in large epicardial arteries. Specifically, FIG. 7 depicts a hemodynamic model using an electrical analog wherein the phenomena described herein is related to assessing a stenosis. The model depicts native fluid flow past a catheter and a stenosis, as well as collateral native fluid flow, to a point distal to the stenosis at which the pressure is identified as P_{distal}. The model also shows the native flow resistance due to the catheter (Cath-R), the native flow resistance due to the stenosis (Stenosis-R), and the native flow resistance along the collateral channel (Collateral-R).

The model is explained with reference to system 500 as a non-limiting example. In some embodiments, distal end 506 is disposed near the proximal end of the stenosis. Using methods described above, infusate is delivered at one or more fluid flow rates to permit the determination of flow rate at which the pressure at the fluid delivery site is determined to be at or sufficiently close to the proximal arterial reference pressure (corresponding to the equipoise flow rate), such as when the pressure measured by pressure guidewire 508 (represented as P_{cofi} in FIG. 7) is the same as the pressure measured by pressure sensor 526. The flow rate through the stenosis may be represented as flow rate Qₚᵤₘₚ,ₑ and may be maintained as pressure guidewire 508 is advanced to the distal end of the stenosis. Once pressure guidewire 508 is at the distal end of the stenosis, pressure P_{discal} may be measured by pressure guidewire 508 as the infusate continues to be delivered at flow rate Qₚᵤₘₚ,ₑ. The resistance of the stenosis may then be calculated using (P_{cofi} - P_{distal})/Q_{pump,e}.

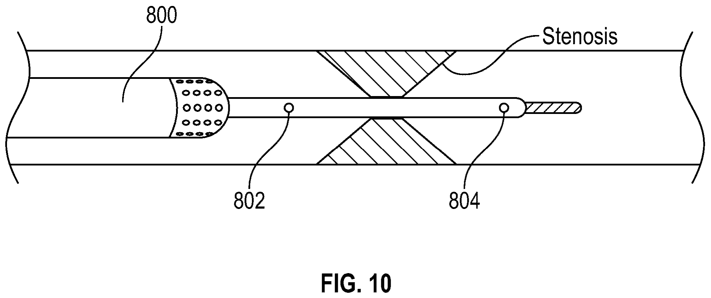

In other embodiments, such as the configuration depicted in reference to FIG. 10, pressure P_{cofi} and pressure P_{distal} may be determined simultaneously by pressure sensor 802 and pressure sensor 804 as infusate is being delivered at flow rate Q_{pump,e}.

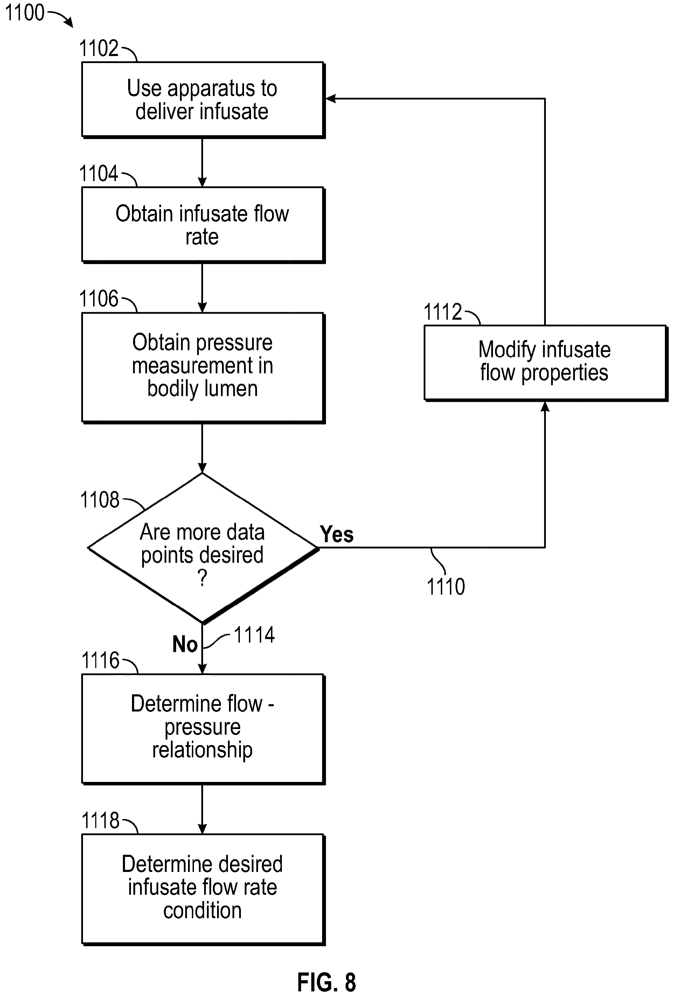

In FIG. 8, a flow chart describing the use of the system 500 in accordance with the present invention is described. System 500 may be used to determine an infusate rate corresponding to a proximal arterial reference pressure that is known or measured in a patient. Method 1100 begins at step 1102, in which an infusion catheter of system 500 is inserted into a vessel to deliver infusate to the patient. At step 1104, an initial infusate flow rate is selected, which may be accomplished using controller 518 to set the volumetric flow rate at which pump 516 delivers infusate through infusion catheter 502. At step 1106, one or more readings are taken inside the patient's vessel, such as with pressure sensor or transducer located on catheter or guidewire, to determine a pressure of the flow at the treatment site. At step 1108, a determination is made whether additional data points are desired. For a linear regression analysis, data for at least two infusate volumetric flow rates are required, but data for additional flow rates may be desired for redundancy or safety purposes, or if data already collected are in close proximity to each other. If additional data is desired, the process continues along path 1110 to step 1112, where the infusate flow properties are modified, e.g., by selecting a higher volumetric flow rate. This process proceeds to steps 1102-1106, at which infusate is delivered to the patient at additional flow rates and pressure values are measured. The foregoing process continues until it is determined that sufficient data points exist at step 1108, after which a flow-pressure relationship is determined to enable computation of equipoise flow rates, such as by using a linear regression analysis, at steps 1116 and 1118, as discussed above. The infusate flow rate corresponding to the proximal arterial reference pressure may thus be determined.

It will be appreciated by those of skill in the art that apparatus and methods described herein advantageously may be used to determine a fractional flow reserve (FFR). FFR may be computed as the instantaneous ratio of pressures in a vessel across a hemodynamic resistance, such as a stenosis, at a specific volumetric flow rate. Advantageously, this value is an instantaneous and absolute value that is microvascular flow dependent, unlike traditional FFR methods that obtain a single FFR value corresponding to "maximal hyperemia," which is a highly arbitrary value that may vary from patient to patient, or exhibit variability within the same patient over time if changes in hyperemic flow occur.

Coronary flow reserve (CFR) is the ratio of resting coronary vascular flow at a baseline condition compared to the coronary flow at full adenosine maximal microvascular dilation. CFR may be obtained by capturing data at the time the infusate flow is initiated. Progressive distal hypoxia occurs when an infusate fluid has little or no oxygen content, which subsequently elicits a natural ischemic vasodilatory response in the patient. Accordingly, this reaction permits the process described in FIG. 8 to be repeatedly performed, without the need for vasodilators such as adenosine or others that currently are used, and are essential to conventional techniques for determining CFR.

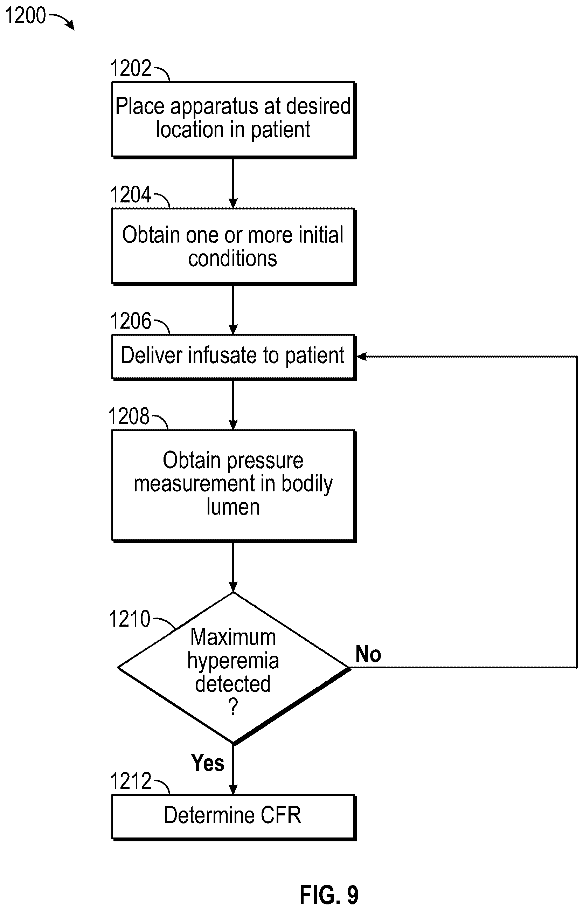

In accordance with yet another aspect of the present invention, CFR may be determined as described in FIG. 9. Method 1200 for determining CFR involves placing the infusion catheter of system 500 at a desired location in a patient's vessel or organ, such as within an artery or other lumen, at step 1202. At step 1204, one or more initial conditions may be obtained, such as obtaining a pressure measurement within a patient's lumen before or at the onset of treatment. Preferably, step 1204 occurs prior to or shortly after infusate is delivered to the patient by pump 516. At step 1206, an infusate is delivered to the patient through the infusion catheter, the infusate preferably having little or no oxygen content to induce ischemia. It is generally well understood that when perfused with a fluid having little or no free oxygen, the resulting ischemia will cause the vessel to undergo vasodilation, typically within 3 to 7 seconds. At step 1208, the pressure in the vessel is monitored to determine when the pressure reaches an asymptotic low value. The flow rate then may be increased and pressure measured; these steps are repeated until no further change in pressure is detected and an equipoise flow rate is established, as described with respect to FIG. 8. This condition has been shown to occur in 7 to 10 seconds. When the equipoise flow rate is determined, this will correspond to the flow rate at maximum hyperemia, at step 1210. At step 1212, CFR then may be determined as the ratio of the initial pressure determined at step 1204 to the pressure measured at the equipoise infusion flow rate at maximum hyperemia.

One of skill in the art will recognize that method 1200 may be performed with or without an occlusion balloon. Advantageously, if apparatus with no occlusion balloon is utilized for method 1200, a practitioner may access bodily lumens having a narrower diameter, thus allowing diagnosis in small spaces previously unavailable as they are too small for balloon utilization. Additionally, assessing the parameters without an occlusion balloon is a safer procedure since there is no balloon-artery contact which may induce dissections, tears, or vessel occlusion.

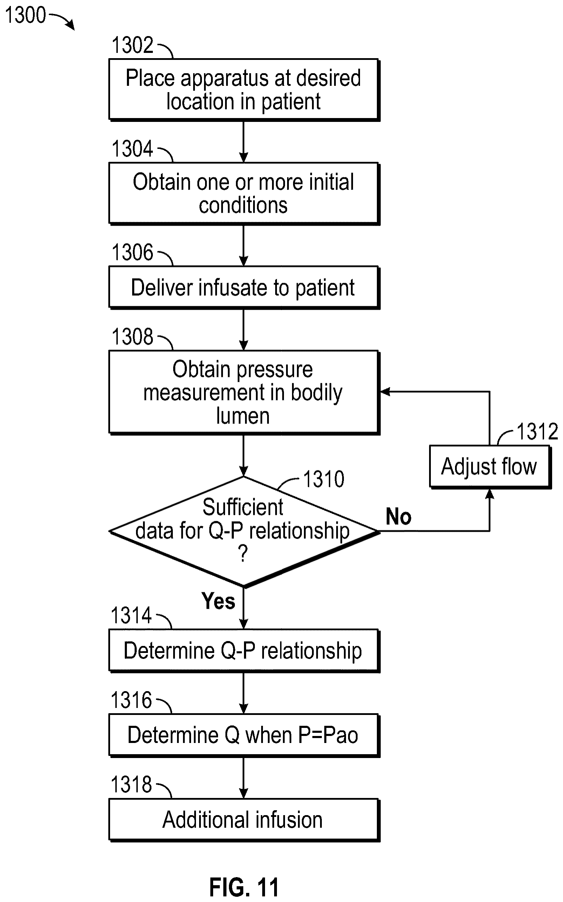

Determination of stenosis resistance or FFR in large epicardial arteries in accordance with principles of the present invention is described with respect to FIG. 10 and the flow chart of FIG. 11. Method 1300 for determining stenosis resistance or FFR involves placing an infusion catheter at a desired location in a patient, such as within an artery or other lumen, at step 1302. Preferably, the infusion catheter is placed such that one or more pressure sensors are located proximal and distal to a treatment site. By way of example, in FIG. 10 shows distal end 800 of an infusion catheter disposed across stenosis such that distal pressure sensor 802 is disposed proximal to the stenosis and distal pressure sensor 804 is disposed distal to stenosis. Referring again to FIG. 11, at step 1304, one or more initial conditions may be obtained, such as pressures distal and proximal to the stenosis obtained by sensors 802 and 804. Preferably, step 1304 occurs prior to or shortly after infusate begins to be delivered to the patient. Alternatively, a pressure sensing wire may be withdrawn to a position antegrade to the stenosis while recording, a process that permits the hemodynamic pressure gradient across the stenosis to be assessed at known infused flow rates.

At step 1306, an infusate is delivered to the patient that preferably has little or no oxygen content. For example, an initial flow value may be selected of 10 ml/minute. Pressure in the vessel then is measured at step 1308. At step 1320, a determination is made whether there is sufficient data to determine a linear relationship between the pressure and the infusate flow rate. If there is insufficient data, the method proceeds to step 1312 and the flow rate of the infused fluid is adjusted. Pressure in the vessel corresponding to the adjusted flow rate is measured at step 1308. The method then proceeds back to step 1310 where the cycle may continue until a determination is made that sufficient data has been obtained and no need for additional pressure measurements is needed at other flow rates, as determined at step 1310. A regression of the accumulated data values of flow and pressure may be performed, at step 1314, and an equipoise flow rate may be determined at step 1316. At step 1318, an additional infusion may be performed at the equipoise flow rate, and FFR may be calculated as the ratio of the pressures measured at each end of the stenosis and stenosis resistance may be calculated as the ratio of the pressure gradient across the stenosis divided by the equipoise flow rate.

In preferred embodiments, flow rates may progress in a step-wise fashion across a range of parameters, for example 0 to 40 ml/minute or more in 10 ml/minute increment steps, though it will be appreciated that the steps may, but need not, be increased at the same rate and indeed may be adjusted arbitrarily. As previously indicated, linearity of the infused flow-resulting pressure relationship can be used to calculate the equipoise value, with as few as 2 infusion levels

One of skill in the art will recognize that method 1300 may be performed with or without an occlusion balloon. It will be further understood that data obtained via method 1300 may be aggregated or shared across multiple patients or multiple procedures to improve accuracy.

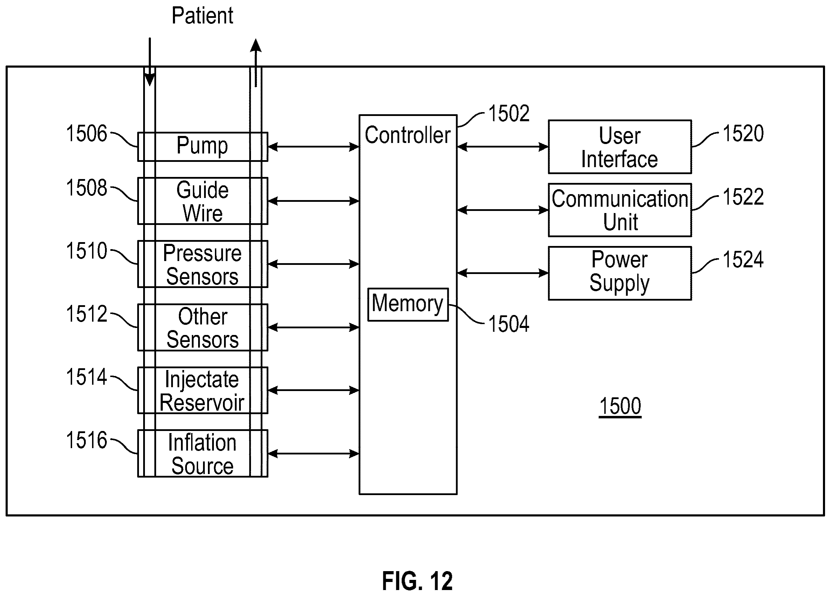

Referring now to FIG. 12, a block diagram of a system constructed in accordance with the present invention is described. For example, system 1500 includes components that may be incorporated in system 100 of FIG. 1 herein. System 1500 includes controller having memory 1504. Controller may be a computer and memory may be a disk drive or flash memory. Controller 1502 is in electrical communication with pump 1506, steerable guide wire 1508, one or more pressure sensors 1510, and optionally other sensors 1512, such as temperature sensors. Controller 1502 is also in communication with injectate reservoir 1514 and inflation source 1516, which may be used to inflate one or more balloons, if present. Pump 1506, guide wire 1508, pressure sensors 1510, optional other sensors 1512, injectate reservoir, and inflation source 1516 are operatively coupled to an infusion catheter inserted into patient.

Controller 1502 is also in communication with user interface 1520, communication unit 1522, and power supply 1524. Controller 1502 includes a processor that is programmed to perform the regression analysis on accumulated data to compute the equipoise infusion rate, as described above. User interface 1520 may include keyboard, mouse device, display screen, touch screen, or other user interface devices. Communication unit 1522 may include alarm, WiFi, Internet, cloud storage, and telecommunication devices. Power supply 1524 may include alternating current power or direct current power or may be switchable therebetween. It will be understood that methods and/or algorithms of the present invention may be stored as programmed instructions, or as non-transitory computer-readable media, accessible to a processor of controller 1502, thereby allowing programmed methods of the present invention, as described above, to be performed in a computer-controlled system.

It is to be understood that the implementations described herein are illustrative and that the scope of the present invention is not limited to those specific embodiments; many variations, modifications, additions, and improvements are possible. For example, functionality may be separated or combined in blocks differently in various embodiments of the disclosure or described with different terminology. These and other variations, modifications, additions, and improvements may fall within the scope of the disclosure as defined in the claims that follow.
The invention is further defined by the following items:
1. A method of determining one or more vascular physiological parameters for a patient, the method comprising:
   advancing a catheter having a lumen into an arterial vessel of the patient, the arterial vessel having antegrade blood flow and the lumen having a distal end;
   measuring a reference arterial pressure at a location proximal to the distal end of the lumen;
   delivering a fluid at a first volumetric flow rate through the lumen into the arterial vessel;
   measuring a first arterial pressure in the arterial vessel, the first arterial pressure corresponding to the first volumetric flow rate;
   delivering the fluid at a second volumetric flow rate through the lumen into the arterial vessel;
   measuring a second arterial pressure in the arterial vessel, the second arterial pressure corresponding to the second volumetric flow rate;
   determining, based on the first volumetric flow rate, the first arterial pressure, the second volumetric flow rate, and the second arterial pressure, an equipoise volumetric flow rate of the fluid at which the pressure proximate to the distal end of the lumen corresponds to the reference arterial pressure.
2. The method of item 1, further comprising determining microvasculature resistance based on the reference arterial pressure and the equipoise volumetric flow rate.
3. The method of item 2, wherein the first arterial pressure corresponds to one of a first peak systolic pressure, a first peak diastolic pressure, a first RMS pressure, or a first mean pressure, and wherein the second arterial pressure corresponds to one of a second peak systolic pressure, a second peak diastolic pressure, a second RMS pressure, or a second mean pressure.
4. The method of item 1, wherein the fluid contains little or no free oxygen.
5. The method of item 1, wherein equipoise volumetric flow rate is determined using a regression analysis.
6. The method of item 1, wherein a stenosis is disposed in the arterial vessel and further comprising:
   delivering the fluid at the equipoise volumetric flow rate through the lumen into the arterial vessel;
   measuring a third arterial pressure proximal of the stenosis; and
   measuring a fourth arterial pressure distal of the stenosis.
7. The method of item 6, further comprising characterizing the stenosis based on the third arterial pressure and the fourth arterial pressure.
8. The method of item 6, further comprising determining a stenosis resistance based on the third arterial pressure, the fourth arterial pressure, and the equipoise volumetric flow rate.
9. The method of item 6, further comprising determining a fractional flow reserve based on the ratio of the fourth arterial pressure to the third arterial pressure.
10. The method of item 1, wherein the fluid is delivered from the catheter into the arterial vessel through an outlet port at a distal end of the catheter.
11. The method of item 1, wherein fluid is delivered from the catheter into the arterial vessel via a plurality of holes disposed in a distal end of the catheter.
12. The method of item 1, wherein the catheter is balloonless.
13. The method of item 1, wherein the reference arterial pressure is a pressure on the proximal section of the catheter.
14. The method of item 1, wherein the reference arterial pressure is aortic pressure.
15. Apparatus for assessing a patient with a vascular stenosis and/or dysfunction, the apparatus comprising:
   a catheter having a distal region sized and shaped to be advanced into an arterial vessel, the catheter comprising a lumen for delivering a fluid into the arterial vessel;
   a pressure sensor disposed at the distal region of the catheter to measure a pressure;
   a reference pressure sensor configured to measure a reference pressure at a location proximal to the distal region; and
   a controller operatively coupled to the reference pressure sensor and the pressure sensor, the controller configured to:
      cause the fluid to be delivered at a first volumetric flow rate through the lumen into the arterial vessel;
      measure a first arterial pressure in the arterial vessel while the fluid is delivered at the first volumetric flow rate;
      cause the fluid to be delivered at a second volumetric flow rate through the lumen into the arterial vessel;
      measure a second arterial pressure in the arterial vessel while the fluid is delivered at the second volumetric flow rate; and
      determine an equipoise volumetric flow rate at which the pressure corresponds to the reference pressure.
16. The apparatus of item 15, wherein the controller is further configured to compute microvasculature resistance by dividing the reference pressure by the equipoise volumetric flow rate.
17. The apparatus of item 15, wherein the fluid contains little or no available oxygen.
18. The apparatus of item 15, wherein the pressure sensor is disposed on the catheter.
19. The apparatus of item 15, wherein the pressure sensor is disposed on a guidewire coupled to the catheter.
20. The apparatus of item 15, wherein the catheter is balloonless.
21. The apparatus of item 15, wherein the controller is further configured to determine the equipoise volumetric flow rate using regression analysis.
22. The apparatus of item 15, wherein the controller is further configured to cause the fluid to be delivered at the equipoise volumetric flow rate.
23. The apparatus of item 22, wherein the pressure sensor is configured to be advanced from a proximal side of a stenosis to a distal side of the stenosis.
24. The apparatus of item 22, further comprising a second pressure sensor, the pressure sensor and second pressure sensor configured to be disposed on opposite sides of a stenosis.
25. The apparatus of item 15, wherein the catheter is configured to deliver the fluid through an outlet port disposed at the distal region.
26. The apparatus of item 15, wherein the catheter is configured to deliver the fluid through a plurality of holes disposed at the distal region.
27. The apparatus of item 15, wherein the reference pressure sensor is disposed on the catheter at a location proximal to the distal region.
28. The apparatus of item 15, wherein the reference pressure sensor is configured to be disposed in the patient's aorta.
29. The apparatus of item 22, further configured to measure a third arterial pressure and a fourth arterial pressure, the third arterial pressure measured at a point proximal of a stenosis in the arterial vessel and the fourth arterial pressure measured at a point distal to the stenosis.
30. The apparatus of item 29, wherein the controller is further configured to characterize the stenosis based on the third arterial pressure and the fourth arterial pressure.
31. The apparatus of item 29, wherein the controller is further configured to determine a stenosis resistance based on the third arterial pressure, the fourth arterial pressure, and the equipoise volumetric flow rate.
32. The apparatus of item 29, wherein the controller is further configured to determine a fractional flow reserve based on the ratio of the fourth arterial pressure to the third arterial pressure.

## Claims

1. A non-transitory processor-readable medium storing code representing instructions to be executed by a processor, the code comprising code to cause the processor to:
receive a reference arterial pressure measured within an arterial vessel of a patient;
determine a first volumetric flow rate;
receive a first arterial pressure measured within the arterial vessel, the first arterial pressure corresponding to the first volumetric flow rate;
determine a second volumetric flow rate;
receive a second arterial pressure measured within the arterial vessel, the second arterial pressure corresponding to the second volumetric flow rate;
determine, based on the first volumetric flow rate, the first arterial pressure, the second volumetric flow rate, and the second arterial pressure, an equipoise volumetric flow rate at which a corresponding pressure corresponds to the reference arterial pressure.

2. The non-transitory processor-readable medium of claim 1, further comprising code to cause the processor to determine microvasculature resistance based on the reference arterial pressure and the equipoise volumetric flow rate.

3. The non-transitory processor-readable medium of claim 2, wherein the first arterial pressure corresponds to one of a first peak systolic pressure, a first peak diastolic pressure, a first RMS pressure, or a first mean pressure, and wherein the second arterial pressure corresponds to one of a second peak systolic pressure, a second peak diastolic pressure, a second RMS pressure, or a second mean pressure.

4. The non-transitory process-readable medium of claim 2, wherein the code to cause the processor to determine microvascular resistance includes code to cause the processor to compute the microvascular resistance by dividing the reference pressure by the equipoise volumetric flow rate.

5. The non-transitory processor-readable medium of claim 1, wherein the equipoise volumetric flow rate is determined using a regression analysis.

6. The non-transitory processor-readable medium of claim 5, wherein the regression analysis includes extrapolation.

7. The non-transitory processor-readable medium of claim 5, wherein the regression analysis includes interpolation.

8. The non-transitory processor-readable medium of claim 5, wherein the regression analysis is linear regression analysis and includes extrapolation when the first arterial pressure and the second arterial pressure are below the reference pressure.

9. The non-transitory processor-readable medium of claim 5, wherein the regression analysis is linear regression analysis and includes interpolation when the first arterial pressure is below the reference pressure and the second arterial pressure is above the reference pressure.

10. The non-transitory processor-readable medium of claim 5, wherein the code to cause the processor to determine the equipoise volumetric flowrate includes code to cause the processor to determine the equipoise volumetric flowrate based on a plurality of infused flow rates including only the first flow rate and the second flow rate.

11. The non-transitory processor-readable medium of claim 5, wherein the code to cause the processor to determine the equipoise volumetric flowrate includes code to cause the processor to determine the equipoise volumetric flowrate based on a plurality of measured pressures including only the first arterial pressure, the second arterial pressure, and the reference pressure.

12. The non-transitory processor-readable medium of claim 1, the code further comprising code to cause the processor to:
receive a third arterial pressure measured proximal to a stenosis within the arterial vessel;
receive a fourth arterial pressure measured distal to the stenosis; and
characterize the stenosis based on the third arterial pressure and the fourth arterial pressure.

13. The non-transitory processor-readable medium of claim 1, the code further comprising code to cause the processor to:
determine a stenosis resistance based on the third arterial pressure, the fourth arterial pressure, and the equipoise volumetric flow rate.

14. The non-transitory processor-readable medium of claim 1, the code further comprising code to cause the processor to:
determine a fractional flow reserve based on the ratio of the fourth arterial pressure to the third arterial pressure.
